# EUROPÄISCHE PATENTSCHRIFT

(11) **EP 1 195 169 B1**
(45) Veröffentlichungstag und Bekanntmachung des Hinweises auf die Patenterteilung: **02.03.2005**
(21) Anmeldenummer: 00121926.0
(22) Anmeldetag: 09.10.2000
(51) Int. Cl.: A61L 9/03, A01M 1/20

(54) **Vorrichtung zum Verdampfen von flüchtigen Substanzen, insbesondere von Insektiziden und/oder Duftstoffen**
Vaporisation device for volatile compounds, in particular for insecticides or perfumes
Diffuseur de produits volatils notamment des insecticides et des parfums

(43) Veröffentlichungstag der Anmeldung: 10.04.2002
(73) Patentinhaber: C.T.R., Consultoria, Técnica e Representaçoes Lda, 2715-251 Almargem do Bispo (PT)
(72) Erfinder: Queiroz Vieira, Pedro, 2775-178 Parede (PT)
(74) Vertreter: Liebl, Thomas, Dipl.-Ing.

(56) Entgegenhaltungen:
- EP-A- 0 362 397
- EP-A- 0 451 331
- EP-A- 0 591 537
- EP-A- 0 998 947
- DATABASE WPI Section Ch, Week 198338 Derwent Publications Ltd., London, GB; Class L03, AN 1983-767651 XP002162690 & JP 58 135183 A (NIPPONDENSO CO LTD), 11. August 1983 (1983-08-11)

## Beschreibung

Die Erfindung betrifft eine Vorrichtung zum Verdampfen von flüchtigen Substanzen, insbesondere von Insektiziden und/oder Duftstoffen, gemäß dem Oberbegriff des Anspruchs 1.

Derartige Vorrichtungen zum Verdampfen sind allgemein bekannt. So sind beispielsweise Verdampfungsvorrichtungen bekannt, bei denen eine in eine Verdampfungsvorrichtung eingesetztes und mit einem Wirkstoff imprägniertes Plättchen erhitzt wird, um den Wirkstoff zu verdampfen. Weiter ist es auch bekannt, in ein Gehäuse einer Verdampfungsvorrichtung einen eine flüchtige Substanz enthaltenden Behälter einzusetzen. Dieser Behälter umfasst einen Docht, der die zu verdampfende Substanz mittels der Kapillarwirkung aus dem Behälter fördert, wobei das aus dem Behälter ragende Dochtende benachbart zu einem Heizelement, wie z. B. einem Keramikblock, angeordnet ist, so dass die Substanz durch die vom Keramikblock abgestrahlte Wärme verdampft und über Lüftungsschlitze im Gehäuse aus diesem in die Umgebung entweichen kann.

So ist beispielsweise aus der gattungsgemäßen EP 0 943 344 A1 eine Verdampfungsvorrichtung für flüchtige Substanzen, insbesondere von Insektiziden und/oder Duftstoffen, bekannt, die ein Gehäuse mit einer darin angeordneten Heizeinrichtung aufweist. Die Heizeinrichtung umfasst einen Heizblock aus Keramik, in dem zur Erwärmung des Heizblocks ein elektrisches Widerstandselement aufgenommen ist, das über elektrische Leitungen mit einem am Gehäuse angeordneten Anschlussstecker gekoppelt ist. Ferner umfaßt diese Vorrichtung einen mit dem Gehäuse verbindbaren Behälter für eine zu verdampfende Substanz, wobei in den Behälter ein Docht einsetzbar ist, der bei mit dem Gehäuse verbundenen Behälter zur Verdampfung der sich im Behälter befindlichen Substanz mit einem aus dem Behälter ragenden Dochtende der Heizeinrichtung zugeordnet ist.

Konkret besteht die Verdampfungsvorrichtung hier aus einem einen Anschlussstecker aufweisenden Steckerteil. Das Steckerteil weist ein Gewinde auf und wird in das Gehäuse eingesetzt, in das auch der Behälter für die zu verdampfenden Substanzen eingesetzt wird. Am Gehäuse sind Stiftausnehmungen vorgesehen, in die die Raststifte so eingesetzt werden, dass diese in das Gewinde des Steckerteils eingreifen. Dadurch kann der Abstand der mit dem Steckerteil verbundenen Heizeinrichtung relativ zu einem aus dem Behälter ragenden Dochtende durch Verdrehen des Steckerteils verändert werden. In einer Ausgestaltung hierzu kann das Steckerteil zudem exzentrisch im Gehäuse gelagert sein, so dass auch hierüber der Relativabstand zwischen dem Dochtende und der Heizeinrichtung je nach dem gewünschten Verdampfungsgrad verändert werden kann. Die Heizeinrichtung ist hier durch einen Keramikblock gebildet, in den ein elektrischer Widerstand einzementiert ist. Als Widerstandselemente sind hier allgemein im Handel erhältliche relativ groß bauende Widerstände verwendet, was einen insgesamt relativ großbauenden Aufbau der Heizeinrichtung und damit entsprechend auch des die Heizeinrichtung umgebenden Gehäuses und der Verdampfungsvorrichtung insgesamt bedingt. Derartige großbauende Verdampfungsvorrichtungen sind optisch weniger ansprechend und können den optischen Gesamteindruck, z. B. in einem Wohnraum, stören. Weiter begünstigt ein derartiger Aufbau mit einer Vielzahl von Bauteilen ebenfalls große Baugrößen, wobei der Aufbau mit einer Vielzahl von Bauteilen zudem auch relativ aufwendig und kompliziert ist, so dass der Herstellaufwand und damit die Verdampfungsvorrichtungen insgesamt relativ teuer sind. Weiter kann mit derartigen Heizelementen insbesondere aufgrund der Verwendung handelsüblicher Widerstandselemente die Verdampfungstemperatur regelmäßig nur in unzureichender Weise genau auf die Zusammensetzung der jeweils zu verdampfenden Substanz abgestimmt werden, was z.B. negative Auswirkungen auf die Entflammbarkeit bestimmter Teile oder der gesamten Verdampfungsvorrichtung und ggf. auch auf den Verdampfungsgrad haben kann.

Ein ähnlicher Aufbau mit den eben genannten Nachteilen ist auch aus der WO 98/58692 und der EP 0 962 132 A1 bekannt.

Es ist daher Aufgabe der Erfindung, eine alternative Vorrichtung zum Verdampfen von flüchtigen Substanzen, insbesondere von Insektiziden und/oder Duftstoffen zu schaffen, die mit kleinen Abmessungen und ohne großen Bauteilaufwand einfach und preiswert herstellbar ist und die bezüglich der Verdampfungstemperatur genau auf die jeweils zu verdampfende Substanz abgestimmt ist.

Diese Aufgabe wird gelöst mit den Merkmalen des Anspruchs 1.

Erfindungsgemäß weist das elektrische Widerstandselement zur Ausbildung einer Heizeinrichtung mit geringen Abmessungen und damit einer insgesamt miniaturisierten Vorrichtung zum Verdampfen von flüchtigen Substanzen einen stabförmigen Widerstandskörper auf, der wenigstens bereichsweise mit einer Widerstandsschicht beschichtet ist, die zur Einstellung eines bestimmten Widerstandswertes entsprechend einer auf die Zusammensetzung der jeweils zu verdampfenden Substanz abgestimmten Verdampfungstemperatur bereichsweise eingeschnitten und/oder bereichsweise eingeschliffen ist.

Vorteilhaft kann ein derartiges Widerstandselement für Heizeinrichtungen von Verdampfungsvorrichtungen relativ kleinbauend ausgebildet werden, so dass auch der Heizblock und die Heizeinrichtung und damit auch das die Heizeinrichtung aufnehmende Gehäuse insgesamt relativ kleinbauend ausgebildet werden können. Dadurch kann beim gleichzeitigen Einsatz eines entsprechend angepassten kleinvolumigen Behälters in das Gehäuse eine Verdampfungsvorrichtung mit geringen Abmessungen, d. h. eine miniaturisierte Verdampfungsvorrichtung geschaffen werden. Eine derartige miniaturisierte Verdampfungsvorrichtung ist aufgrund des reduzierten Material- und Bauteilaufwands zudem auch relativ einfach und damit preiswert herstellbar, z. B. als einmal benutzbarer Wegwerfartikel.

Ein weiterer besonderer Vorteil einer derartigen Verdampfungseinrichtung ist, dass die Verdampfungstemperatur mit einem derartigen Widerstandselement, bei der die Widerstandsschicht zur Einstellung eines bestimmten Widerstandswertes bereichsweise eingeschnitten und/oder bereichsweise eingeschliffen ist, optimal auf die Zusammensetzung der jeweils zu verdampfenden Substanz abgestimmt werden kann. Dadurch wird z.B. vorteilhaft die Gefahr der Entflammbarkeit der Vorrichtung insgesamt oder bestimmter Bestandteile reduziert und zudem auch eine ggf. negative Auswirkung auf den Verdampfungsgrad vermeidbar.

Grundsätzlich gibt es verschiedene Möglichkeiten die Widerstandsschicht zur Einstellung eines bestimmten Widerstandswertes einzuschneiden bzw. einzuschleifen. Gemäß einer bevorzugten Ausführungsform nach Anspruch 2 wird die Widerstandsschicht um den stabförmigen, vorzugsweise zylindrischen Widerstandskörper herum spiralförmig eingeschnitten, vorzugsweise durch Laserspiralschneiden. Mit einem derartigen spiralförmigen Einschnitt kann der Widerstandswert für eine optimale Verdampfungsleistung auf besonders einfache Weise sehr genau eingestellt werden.

Die Widerstandsschicht kann grundsätzlich ebenfalls aus unterschiedlichen Materialien hergestellt sein, so z. B. in Form einer Edelmetallschicht. In einer gemäß Anspruch 3 besonders bevorzugten Ausführungsform ist die Widerstandsschicht jedoch als eine Metalloxidschicht ausgebildet, vorzugsweise eine Nickel-Chromlegierungsschicht. Eine derartige Metalloxidschicht wird vorteilhaft thermochemisch aufgebrannt, z. B. als Dünnschicht im Vakuum aufgedampft bzw. gesputtert. Nach der Aufbringung der Widerstandsschicht wird diese vorzugsweise einem thermischen Verfahren unterworfen, um die Widerstandsschicht zu stabilisieren.

Zusätzlich oder alternativ dazu kann der Widerstandskörper aus Keramik hergestellt sein, vorzugsweise mit einem hohen Gehalt an Al₂O₃ (Aluminiumoxid), wodurch eine besonders gute Wärmeleitfähigkeit des Widerstandskörpers und damit des Widerstandselementes insgesamt erreichbar ist. Der Gehalt an Al₂O₃ ist abhängig von den jeweils konkret gegebenen Einbauverhältnissen, z. B. dem verwendeten Gehäusematerial, Dochtmaterial, etc.

Gemäß Anspruch 4 sind auf die Enden des beschichteten stabförmigen Widerstandskörpers jeweils Metallkappen aufgesetzt, die vorzugsweise aufgepresst sind. An diesen Kappen ist jeweils eine elektrische Leitung angebracht, vorzugsweise angeschweißt, die jeweils mit dem Anschlussstecker gekoppelt sind. Vorzugsweise werden als elektrische Leitungen für eine gute elektrische Leitung Kupferdrähte verwendet. Mit derartigen Metallkappen wird zudem ein guter elektrischer Kontakt zu der Widerstandsschicht auf einfache und funktionssichere Weise hergestellt.

Grundsätzlich gibt es verschiedene Möglichkeiten das stabförmige Widerstandselement am Heizblock anzuordnen. In einer besonders bevorzugten Ausführungsform nach Anspruch 5 ist vorgesehen, dass das stabförmige Widerstandselement in eine Aussparung des Heizblocks einbringbar ist, wobei das Widerstandselement dort mit einem eine hohe Wärmeleitfähigkeit aufweisenden Material vergossen ist, um das Widerstandselement im Heizblock sicher zu fixieren. Das eine hohe Wärmeleitfähigkeit aufweisende Material ist vorzugsweise ein flammenbeständiger Isolationszement. Weiter ist an gegenüberliegenden Aussparungsenden der Aussparung zu beiden Seiten des Widerstandselementes jeweils ein Schlitz ausgebildet, durch die die elektrischen Leitungen aus dem Heizblock zu dem Anschlussstecker hin herausgeführt sind. Mit einem derartigen Aufbau kann das Widerstandselement im Rahmen der Montage auf einfache Weise in die Aussparung eingesetzt werden, z. B. auch mit einem Klemmschluss, so dass das Widerstandselement beim Vergießen nicht mehr verrutschen kann. Weiter können die elektrischen Leitungen mit einem derartigen Aufbau auf einfache Weise auch in Richtung zu dem Anschlussstecker hingebogen werden. Die elektrischen Leitungen können dabei auf herkömmliche Weise isoliert sein.

Ein besonders kompakter Aufbau ergibt sich gemäß Anspruch 6 dadurch, dass die Länge des Widerstandselementes in Aussparungslängsrichtung gesehen in etwa der Breite des Heizblocks entspricht, so dass die im Kappenbereich in etwa rechtwinklig bezüglich des Widerstandselementes abgewinkelten elektrischen Leitungen in etwa parallel zueinander verlaufen sowie in etwa auf einer Linie mit den beiden Steckerverbindungen des Anschlusssteckers liegen. Mit einer derartigen Ausrichtung der elektrischen Leitungen wird ein besonders kompakter Aufbau der Heizeinrichtung erreicht, was ebenfalls dazu beiträgt die Größe des Gehäuses und damit der gesamten Verdampfungsvorrichtung in der erwünschten Weise zu minimieren.

Gemäß Anspruch 7 ist im Heizblock benachbart zum elektrischen Widerstandselement eine Dochtaussparung, vorzugsweise ein Durchgangsloch oder eine randseitig durchgehende Einbuchtung, ausgebildet, in die das Dochtende hineinragt. Mit einer derartigen Dochtaussparung ist eine einfache und funktionssichere Zuordnung des Dochtendes zum Heizblock und damit zur Heizungseinrichtung möglich, was dazu beiträgt, eine effektive Verdampfung zu gewährleisten.

Eine besonders einfache und schnelle Montage der Heizeinrichtung im Gehäuse ist gemäß Anspruch 8 dann möglich, wenn das Gehäuse wenigstens zweiteilig aus einer Oberschale und einer Unterschale aufgebaut ist. Die Oberschale und die Unterschale können beispielsweise durch Rast- und/oder Clipelemente miteinander verbunden werden. In der Unterschale sind vorzugsweise Verbindungsmittel zur Verbindung des Behälters mit dem Gehäuse ausgebildet, z. B. Rastelemente. Wenigstens eine der beiden Schalen weist dabei auch Lüftungsschlitze zum Entweichen der verdampften Substanz in die Umgebung auf. Die Lüftungsschlitze sind dabei vorzugsweise im Bereich oberhalb des Dochtendes in der Oberschale ausgebildet. Ein derartiges zweiteiliges Gehäuse ist auf besonders einfache und preiswerte Weise herstellbar.

Gemäß Anspruch 9 umfasst der Anschlussstecker eine Basisplatte, von der die Steckerstifte einerseits abstehen und zu der andererseits die elektrischen Leitungen geführt sind. Die Basisplatte ist dabei gegebenenfalls mit weiteren im Gehäuse angeordneten Bauteilen gemäß Anspruch 10 für eine feste und damit klapperfreie Fixierung zwischen der Oberschale und der Unterschale verklemmt. Damit wird ein hochwertiger Aufbau möglich, der insgesamt auch sehr stabil ist und eine sichere Fixierung der Bauteile in einer vorbestimmten Position möglich ist.

Um die Verdampfungsvorrichtung vom Gesamteindruck her ästhetischer zu gestalten, ist gemäß Anspruch 11 an der Oberschale ein dekoratives Element befestigbar. Vorzugsweise ist an der Oberschale eine umlaufende Randkante ausgebildet, an der ein derartiges dekoratives Element einrastbar bzw. aufclipsbar ist. Beispielsweise kann ein derartiges dekoratives Element die Form einer Blumenblüte aufweisen.

Die Erfindung wird nachfolgend anhand einer Zeichnung näher erläutert.

Es zeigen:
- Fig. 1: eine schematische, perspektivische Ansicht einer erfindungsgemäßen miniaturisierten Verdampfungsvorrichtung,
- Fig. 2: eine schematische, perspektivische Darstellung der Verdampfungsvorrichtung gemäß Fig. 1 mit abgehobener Oberschale des zweiteiligen Gehäuses,
- Fig. 3: eine schematische, perspektivische Darstellung eines Behälters, eines Dochtes und einer Heizeinrichtung der in den Figuren 1 und 2 dargestellten Verdampfungsvorrichtung,
- Fig. 4: eine vergrößerte Draufsicht auf eine Heizeinrichtung,
- Fig. 5: eine schematische Seitenansicht der Heizeinrichtung gemäß Fig. 4,
- Fig. 6: eine schematische, perspektivische Darstellung der Heizeinrichtung der Fig. 4 und 5,
- Fig. 7: eine vergrößerte, schematische Draufsicht auf ein Widerstandselement mit elektrischen Leitungen,
- Fig. 8: eine schematische Rückansicht des Widerstandselementes gemäß Fig. 7,
- Fig. 9: eine schematische Seitenansicht des Widerstandselementes gemäß Fig. 7,
- Fig. 10: eine schematische, vergrößerte Draufsicht auf einen als Keramikkörper ausgebildeten Heizblock,
- Fig. 11: eine schematische Schnittansicht entlang der Linie A-A der Fig. 10, und
- Fig. 12: eine schematische Seitenansicht der Darstellung gemäß Fig. 10.

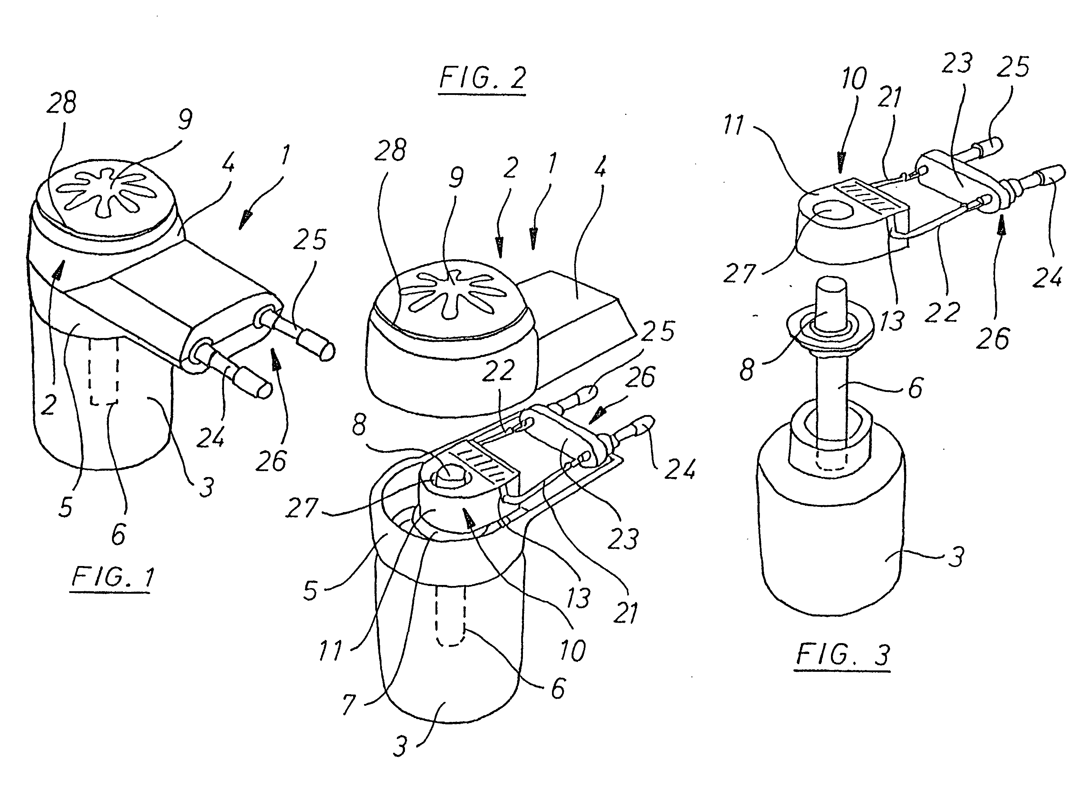

In der Fig. 1 ist schematisch und perspektivisch eine miniaturisierte Verdampfungsvorrichtung 1 dargestellt, die ein Gehäuse 2 und einen damit verbindbaren Behälter 3 für eine zu verdampfende Substanz aufweist. Der Behälter 3 ist z. B. über eine Rastverbindung in das Gehäuse 2 einclipsbar.

Wie dies insbesondere aus der Fig. 2 ersichtlich ist, die die Verdampfungsvorrichtung 1 in einer perspektivischen Darstellung zeigt, ist das Gehäuse 2 zweiteilig aus einer Oberschale 4 und einer Unterschale 5 aufgebaut. Die Oberschale 4 und die Unterschale 5 sind beispielsweise miteinander durch Rast- und/oder Clipelemente lösbar verbunden. Die Unterschale 5 weist hier nicht näher dargestellte Verbindungsmittel zur Verbindung des Behälters 3 mit der Unterschale 5 und damit mit dem Gehäuse 2 auf.

Wie dies insbesondere auch aus der Fig. 3 ersichtlich ist, die die Verdampfungsvorrichtung 1 aus Übersichtlichkeitsgründen ohne das Gehäuse 2 zeigt, ist in dem Behälter 3 ein Docht 6 mittels eines Dochthalters 7 einsetzbar, so dass der Docht 6 im eingesetzten Zustand mit einem Dochtende 8 aus dem Behälter 3 in das Gehäuse 2 ragt, wie dies wiederum aus der Fig. 2 ersichtlich ist. Die Oberschale 4 weist im Bereich oberhalb des Dochtendes 8 Lüftungsschlitze 9 zum Entweichen der verdampften Substanz auf.

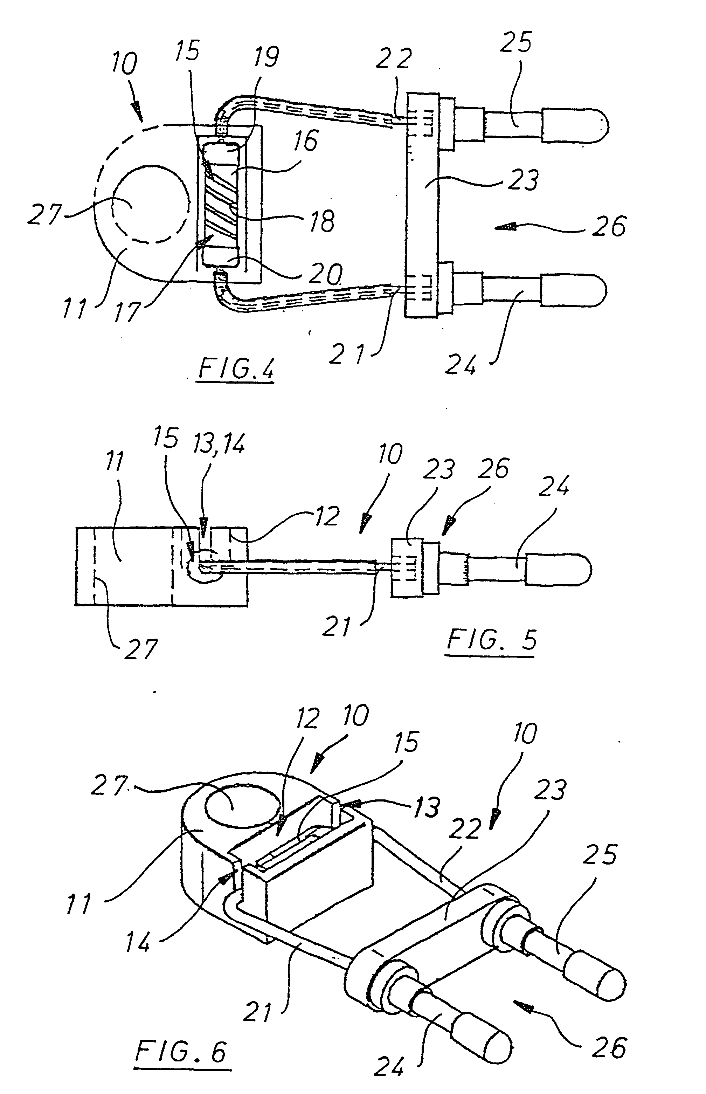

Wie dies aus den Figuren 2 und 3 ferner ersichtlich ist, umfasst die Verdampfungsvorrichtung 1 ferner auch noch eine Heizeinrichtung 10, die in den Figuren 4 bis 6 in unterschiedlichen Darstellungsarten in vergrößerter Weise dargestellt ist.

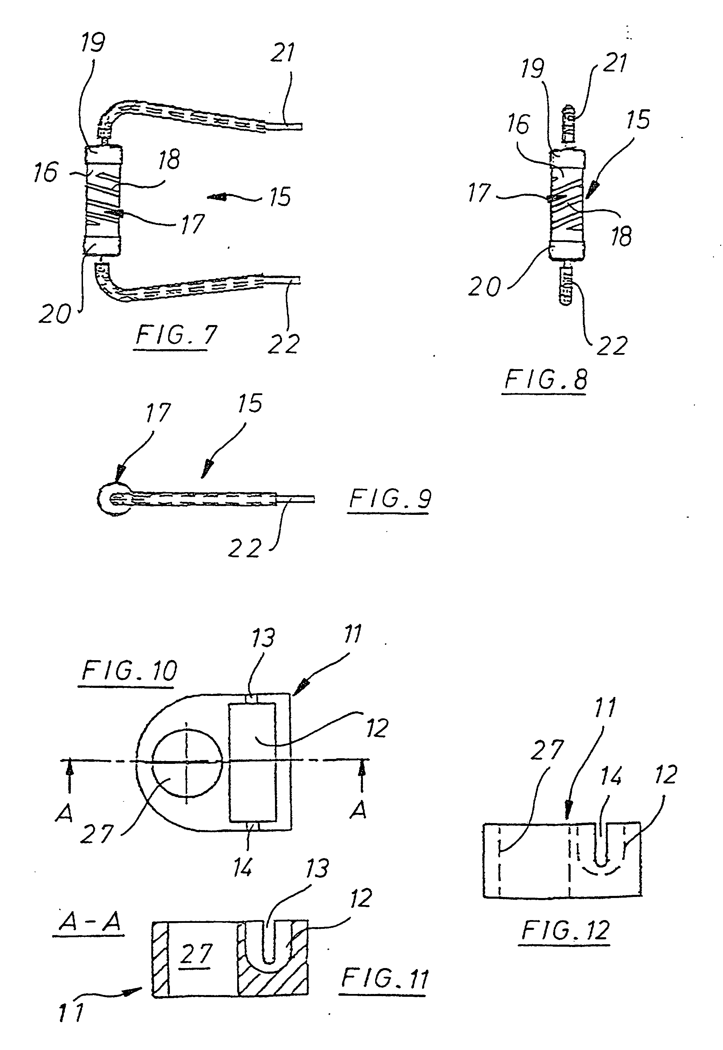

In der Fig. 4 ist eine Draufsicht, in der Fig. 5 eine Seitenansichtung und in der Fig. 6 eine perspektivische Ansicht der Heizeinrichtung 10 gezeigt. Aus diesen Darstellungen wird ersichtlich, dass die Heizeinrichtung 10 einen Keramikblock 11 als Heizblock umfasst, in dem eine Aussparung 12 ausgebildet ist. An in Längsrichtung der Aussparung 12 gegenüberliegenden Aussparungsseitenwänden ist jeweils ein Schlitz 13, 14 ausgebildet. In den Figuren 10 bis 12 ist der Keramikblock 11 in unterschiedlichen Darstellungen gezeigt, so dass dadurch insbesondere die Geometrie der Aussparung 12 sowie der Schlitze 13, 14 ersichtlich wird. So weist die Aussparung 12 im Querschnitt gesehen hier eine lediglich beispielhaft gewählte U-Form auf. Der Keramikblock 11 weist z.B. eine maximale Breite und Länge von ca. 16 mm sowie eine Höhe von ca. 9 mm auf.

In die Aussparung 12 ist ein stabförmiges Widerstandselement 15 eingebracht, das zur Ausbildung einer Heizeinrichtung 10 mit insgesamt geringen Abmessungen aus einem mit einer Widerstandsschicht 16 aus Metalloxid beschichteten Widerstandskörper 17 ausgebildet ist, der hier nicht im Detail dargestellt ist. Zur Einstellung eines bestimmten Widerstandswertes ist die Widerstandsschicht 16 aus Metalloxid, z. B. einer Nickel-Chromlegierungsschicht, spiralförmig, z. B. durch Laserspiralschneiden, eingeschnitten, so dass ein Spiraleinschnitt 18 um den stabförmigen, zylindrischen Widerstandskörper 17 herum ausgebildet ist. In den Figuren 7 bis 9 ist das Widerstandselement 15 in unterschiedlichen Darstellungen ohne Anbindung an den Anschlussstecker 26 gezeigt.

Der Widerstandskörper 17 ist vorzugsweise aus Keramik hergestellt mit einem bestimmten Gehalt an Al₂O₃ zur Einstellung guter Wärmeleiteigenschaften. Beispielsweise weist der Widerstandskörper 17 eine Länge von ca. 10 mm und einen Durchmesser von ca. 3 mm auf. Auf die Enden des beschichteten, stabförmigen, zylindrischen Widerstandskörpers 17 ist für eine elektrische Verbindung zu der Widerstandsschicht 16 jeweils eine Metallkappe 19, 20 aufgesetzt, vorzugsweise aufgepresst. An diesen Kappen 19, 20 ist jeweils eine elektrische Leitung 21, 22, vorzugsweise ein Kupferdraht, angeschweißt, die mit einem Isolationsmaterial isoliert sind.

Wie dies insbesondere aus den Figuren 4 bis 6 ersichtlich ist, wird das Widerstandselement 15 in die Aussparung 12 eingelegt, wobei die beiden elektrischen Leitungen 21, 22 durch die Schlitze 13, 14 aus dem Keramikblock 11 herausgeführt werden. Anschließend wird dann das Widerstandselement 15 mit einem eine hohe Wärmeleitfähigkeit aufweisenden, flammenbeständigen Isolationszement vergossen, um das Widerstandselement 15 im Keramikblock 11 bei nach wie vor gegebener guter Wärmeleitfähigkeit zu fixieren.

Wie dies aus den Figuren 4 bis 6 weiter ersichtlich ist, entspricht die Länge des Widerstandselements 15 in Aussparungslängsrichtung gesehen in etwa der Breite des Keramikblocks 11, so dass die elektrischen Leitungen in etwa rechtwinklig bezüglich des Widerstandselements 15 abgewinkelt und parallel zueinander so wie in etwa auf einer Linie mit den beiden Steckerstiften 24, 25 eines Anschlusssteckers 26 zu einer Basisplatte 23 des Anschlusssteckers 26 geführt sind. Damit ergibt sich ein insgesamt sehr platzsparender und kompakter Aufbau, der eine miniaturisierte Bauweise der Verdampfungsvorrichtung 1, die in den Darstellungen der Figuren 1 bis 2 ungefähr in Originalgröße dargestellt ist, möglich wird.

Wie dies aus der Fig. 10 weiter ersichtlich ist, ist im Keramikblock 11 benachbart zur Aussparung 12 eine kreisförmige Dochtaussparung 27 vorgesehen in die im montierten Zustand der Verdampfungsvorrichtung das Dochtende 8 hineinragt, wie dies insbesondere aus der Fig. 2 ersichtlich ist.

Im montierten Zustand ist zudem die Heizeinrichtung 10 sowie die Basisplatte 23 des Anschlusssteckers 26 zwischen der Oberschale 4 und der Unterschale 5 verklemmt.

Wie dies in den Figuren 1 und 2 lediglich beispielhaft und schematisch dargestellt ist, ist an der Oberschale eine umlaufende Randkante 28 ausgebildet, an der ein dekoratives Element, mit einem entsprechenden Gegenstück einrastbar ist, z. B. ein dekoratives Element in Form einer Blumenblüte, was hier jedoch nicht dargestellt ist.

## Patentansprüche

1. Vorrichtung zum Verdampfen von flüchtigen Substanzen, insbesondere von Insektiziden und/oder Duftstoffen,
mit einem Gehäuse,
mit einer in dem Gehäuse angeordneten Heizeinrichtung, die einen Heizblock vorzugsweise aus Keramik umfasst, in dem zur Erwärmung des Heizblocks ein elektrisches Widerstandselement aufgenommen ist, das über elektrische Leitungen mit einem am Gehäuse angeordneten Anschlussstecker gekoppelt ist,
mit einem mit dem Gehäuse verbindbaren Behälter für eine zu verdampfende Substanz, wobei in den Behälter ein Docht einsetzbar ist, der bei mit dem Gehäuse verbundenem Behälter zur Verdampfung der sich im Behälter befindlichen Substanz mit einem aus dem Behälter ragenden Dochtende der Heizeinrichtung zugeordnet ist,
**dadurch gekennzeichnet,**
**dass** das elektrische Widerstandselement (15) zur Ausbildung einer Heizeinrichtung (10) mit geringen Abmessungen und damit einer insgesamt miniaturisierten Vorrichtung (1) zum Verdampfen von flüchtigen Substanzen einen stabförmigen Widerstandskörper (17) aufweist, der wenigstens bereichsweise mit einer Widerstandsschicht (16) beschichtet ist, die zur Einstellung eines bestimmten Widerstandswertes entsprechend einer auf die Zusammensetzung der jeweils zu verdampfenden Substanz abgestimmten Verdampfungstemperatur bereichsweise eingeschnitten und/oder bereichsweise eingeschliffen ist.

2. Vorrichtung nach Anspruch 1, **dadurch gekennzeichnet, dass** die Widerstandsschicht (16) um den stabförmigen, vorzugsweise zylinderförmigen Widerstandskörper (17) herum spiralförmig mit einem Spiralschnitt (18) eingeschnitten ist, vorzugsweise durch Laserspiralschneiden.

3. Vorrichtung nach Anspruch 1 oder Anspruch 2, **dadurch gekennzeichnet,**
**dass** die Widerstandsschicht (16) eine Metalloxidschicht, vorzugsweise eine Nickel-Chromlegierungsschicht, ist und/oder
**dass** der Widerstandskörper (17) aus Keramik hergestellt ist, vorzugsweise mit einem hohen Gehalt an Al₂O₃.

4. Vorrichtung nach einem der vorhergehenden Ansprüche, **dadurch gekennzeichnet,**
**dass** auf die Enden des beschichteten, stabförmigen Widerstandskörpers (17) jeweils Metallkappen (19, 20) aufgesetzt, vorzugsweise aufgepresst, sind, und
**dass** an diesen Kappen (19, 20) jeweils eine elektrische Leitung (21, 22), vorzugsweise ein Kupferdraht, angebracht, vorzugsweise angeschweißt, ist, die mit dem Anschlussstecker (26) gekoppelt sind.

5. Vorrichtung nach einem der vorhergehenden Ansprüche, **dadurch gekennzeichnet,**
**dass** das stabförmige Widerstandselement (15) in eine Aussparung des Heizblocks (11) einbringbar ist und dort mit einem eine hohe Wärmeleitfähigkeit aufweisenden Material, vorzugsweise einem flammenbeständigen Isolationszement, vergossen ist, und
**dass** an gegenüberliegenden Aussparungswänden zu beiden Seiten des Widerstandselementes (15) jeweils ein Schlitz (13, 14) ausgebildet ist, durch die die elektrischen Leitungen (21, 22) aus dem Heizblock (11) zu dem Anschlussstecker (26) hin herausgeführt sind.

6. Vorrichtung nach Anspruch 5, **dadurch gekennzeichnet,**
**dass** die Länge des Widerstandselements (15) in Aussparungslängsrichtung gesehen in etwa der Breite des Heizblocks (11) entspricht dergestalt,
**dass** die im Kappenbereich in etwa rechtwinklig bezüglich des Widerstandselementes abgewinkelten elektrischen Leitungen (21, 22) in etwa parallel zueinander verlaufen sowie in etwa auf einer Linie mit den beiden Steckerstiften (24, 25) des Anschlusssteckers (26) liegen.

7. Vorrichtung nach einem der vorhergehenden Ansprüche, **dadurch gekennzeichnet, dass** im Heizblock (11) benachbart zum elektrischen Widerstandselement (15) eine Dochtaussparung (27), vorzugsweise ein Durchgangsloch oder eine randseitig durchgehende Einbuchtung, ausgebildet ist, in die das Dochtende (8) hineinragt.

8. Vorrichtung nach einem der vorhergehenden Ansprüche, **dadurch gekennzeichnet,**
**dass** das Gehäuse (2) wenigstens zweiteilig aus einer Oberschale (4) und einer Unterschale (5) aufgebaut ist, die vorzugsweise miteinander durch Rast- und/oder Clipelemente verbindbar sind,
**dass** die Unterschale (5) Verbindungsmittel zur Verbindung des Behälters (3) mit dem Gehäuse (2) aufweist, und
**dass** wenigstens eine der beiden Schalen (4, 5), vorzugsweise die Oberschale (4) im Bereich oberhalb des Dochtendes (8), Lüftungsschlitze (9) zum Entweichen der verdampften Substanz aufweist.

9. Vorrichtung nach einem der vorhergehenden Ansprüche, **dadurch gekennzeichnet, dass** der Anschlussstecker (26) eine Basisplatte (23) umfasst, von der die Steckerstifte (24, 25) einerseits abstehen und zu der andererseits die elektrischen Leitungen (21, 22) geführt sind.

10. Vorrichtung nach Anspruch 8 oder Anspruch 9, **dadurch gekennzeichnet, dass** die im Gehäuse (2) angeordneten Bauteile, vorzugsweise eine gegebenenfalls vorhandene Basisplatte (23) des Anschlusssteckers (26) und/oder die Heizeinrichtung (10) im montierten Zustand zwischen der Oberschale (4) und der Unterschale (5) verklemmt ist.

11. Vorrichtung nach einem der Ansprüche 8 bis Anspruch 10, **dadurch gekennzeichnet, dass** an der Oberschale (4) ein dekoratives Element befestigbar, vorzugsweise an einer umlaufenden Randkante (28) einrastbar ist.

## Claims

1. Device for vaporizing volatile substances, in particular insecticides and/or perfumes,
with a housing,
with a heating arrangement which is disposed in the housing and comprises a heating block, preferably of ceramic, in which, for the purpose of warming the heating block, an electrical resistance element is received which is coupled via electrical lines to a connector plug disposed on the housing, and
with a container for a substance to be vaporized, which container can be connected to the housing and into which container it is possible to insert a wick which, when the container is connected to the housing, has a wick end protruding from the container and assigned to the heating arrangement in order to vaporize the substance located in the container,
**characterized in that**
in order to form a heating arrangement (10) with small dimensions and thus an overall miniaturized device (1) for vaporizing volatile substances, the electrical resistance element (15) has a rod-shaped resistance body (17) which is coated at least in some areas with a resistance layer (16) which, in order to set a defined resistance value corresponding to a vaporization temperature for the composition of the respective substance to be vaporized, is notched in some areas and/or ground-in in some areas.

2. Device according to Claim 1, **characterized in that** the resistance layer (16) around the rod-shaped, preferably cylinder-shaped resistance body (17) is notched in a helical formation with a helical cut (18), preferably by laser helical cutting.

3. Device according to Claim 1 or Claim 2, **characterized in that**
the resistance layer (16) is a metal oxide layer, preferably a nickel-chromium alloy layer, and/or
the resistance body (17) is made of ceramic, preferably with a high content of Al₂O₃.

4. Device according to one of the preceding claims, **characterized in that**
metal caps (19, 20) are in each case fitted, preferably pressed, onto the ends of the coated, rod-shaped resistance body (17), and
an electrical line (21, 22), preferably a copper wire, is in each case applied, preferably welded, to these caps (19, 20), said electrical lines (21, 22) being coupled to the connector plug (26).

5. Device according to one of the preceding claims, **characterized in that**
the rod-shaped resistance element (15) can be introduced into a recess of the heating block (11) and is there encapsulated with a material having high thermal conductivity, preferably a flame-resistant insulation cement, and,
at opposite recess walls, on both sides of the resistance element (15), a slit (13, 14) is in each case formed through which the electrical lines (21, 22) are routed from the heating block (11) to the connector plug (26).

6. Device according to Claim 5, **characterized in that**
the length of the resistance element (15), seen in the longitudinal direction of the recess, corresponds approximately to the width of the heating block (11) in such a way that
the electrical lines (21, 22) angled in the cap area approximately at right angles with respect to the resistance element extend approximately parallel to one another and lie approximately on a line with the two plug pins (24, 25) of the connector plug (26).

7. Device according to one of the preceding claims, **characterized in that** in the heating block (11), adjacent to the electrical resistance element (15), a wick recess (27) is formed, preferably a through-hole or a continuous indentation formed at the edge, into which the wick end (8) protrudes.

8. Device according to one of the preceding claims, **characterized in that**
the housing (2) is made up of at least two parts, namely an upper shell (4) and a lower shell (5), which can preferably be connected to one another by catch elements and/or clip elements,
the lower shell (5) has connector means for connecting the container (3) to the housing (2), and
at least one of the two shells (4, 5), preferably the upper shell (4), has, in the area above the wick end (8), ventilation slots (9) for escape of the vaporized substance.

9. Device according to one of the preceding claims, **characterized in that** the connector plug (26) comprises a base plate (23) from which, on one side, the plug pins (24, 25) protrude and to which, on the other side, the electrical lines (21, 22) are routed.

10. Device according to Claim 8 or Claim .9, **characterized in that** the structural components disposed in the housing (2), preferably an optional base plate (23) of the connector plug (26) and/or the heating arrangement (10), are clamped between the upper shell (4) and the lower shell (5) in the assembled state.

11. Device according to one of Claims 8 to 10, **characterized in that** a decorative element can be secured on the upper shell (4), preferably latched onto a circumferential rim (28).

## Revendications

1. Dispositif de vaporisation de substances volatiles, en particulier d'insecticides et/ou de parfums, pourvu d'un boîtier, avec un dispositif de chauffage disposé dans le boîtier, comprenant un bloc de chauffage de préférence en céramique, dans lequel un élément de résistance électrique couplé au moyen d'un circuit électrique à une fiche de connexion disposée sur le boîtier est installé pour le chauffage du bloc de chauffage, pourvu d'un réservoir pouvant être relié au boîtier pour une substance à vaporiser, une mèche affectée à une extrémité de mèche saillant hors du réservoir de l'installation de chauffage dans le réservoir relié au boîtier pouvant être ajustée dans le réservoir pour la vaporisation de la substance se trouvant dans le réservoir, **caractérisé en ce que** l'élément de résistance électrique (15) comporte un corps de résistance en forme de barreau (17) pour former une installation de chauffage (10) présentant des dimensions inférieures et ainsi un dispositif globalement miniaturisé (1) pour vaporiser des substances volatiles, corps recouvert d'au moins une couche de résistance (16) dans cette zone, qui est entaillée dans cette zone et/ou bouclée dans cette zone pour ajuster une valeur de résistance déterminée correspondant à une température de vaporisation déterminée à partir de la composition de la substance correspondante à vaporiser.

2. Dispositif selon la revendication 1, **caractérisé en ce que** la couche de résistance (16) est entaillée autour du corps de résistance en forme de barreau (17), de préférence cylindrique en forme de spirale avec une section en spirale (18), de préférence au moyen de découpes en spirale au laser.

3. Dispositif selon la revendication 1 ou 2, **caractérisée en ce que** la couche de résistance (16) est une couche d'oxyde métallique, de préférence une couche en alliage de nickel-chrome, et/ou **en ce que** le corps de résistance (17) est fabriqué en céramique, de préférence présentant une teneur élevée en Al₂O₃.

4. Dispositif selon l'une quelconque des revendications précédentes, **caractérisé en ce que** des chapeaux métalliques (19, 20) sont appliqués, de préférence pressés respectivement sur les extrémités du corps de résistance enduit en forme de barreau (17), et **en ce qu'**un circuit électrique (21, 22), de préférence un fil de cuivre, couplé à la fiche de connexion (26) est disposé respectivement sur ces chapeaux (19, 20), de préférence soudé.

5. Dispositif selon l'une quelconque des revendications précédentes, **caractérisé en ce que** l'élément de résistance en forme de barreau (15) peut être disposé dans un évidement du bloc de chauffage (11) et est coulé ici avec un matériau présentant une conductivité thermique élevée, de préférence un ciment isolant résistant aux flammes, et **en ce qu'**une fente (13, 14) au travers de laquelle les circuits électriques (21, 22) sortent du bloc de chauffage (11) vers la fiche de connexion (26) est respectivement conçue sur les parois de l'évidement opposées sur les deux côtés de l'élément de résistance (15).

6. Dispositif selon la revendication 5, **caractérisé en ce que** la longueur de l'élément de résistance (15) vu dans la direction longitudinale de l'évidement correspond à environ la largeur du bloc de chauffage (11), et **en ce que** les circuits électriques (21, 22) courbés dans la zone de chapeau approximativement à angle droit par rapport à l'élément de résistance sont disposés approximativement parallèlement l'un par rapport à l'autre et se trouvent également approximativement sur une ligne avec les deux broches de connexion (24, 25) de la fiche de connexion (26).

7. Dispositif selon l'une quelconque des revendications précédentes, **caractérisé en ce qu'**un évidement de mèche (27), de préférence un orifice de passage ou une anse s'étendant du côté du bord, dans lequel l'extrémité de la mèche (8) fait saillie, est formé dans le bloc de chauffage (11) à proximité de l'élément de résistance électrique (15).

8. Dispositif selon l'une quelconque des revendications précédentes, **caractérisé en ce que** le boîtier (2) est conçu au moins en deux parties d'une coque supérieure (4) et d'une coque inférieure (5), pouvant être reliées par des éléments de clip et/ou à crans, **en ce que** la coque inférieure (5) comporte des moyens de liaison pour relier le réservoir (3) au boîtier (2), et **en ce qu'**au moins une des deux coques (4, 5), de préférence la coque supérieure (4) dans la zone au dessus de l'extrémité de la mèche (8), comporte des fentes d'aération (9) pour l'échappement de la substance vaporisée.

9. Dispositif selon l'une quelconque des revendications précédentes, **caractérisé en ce que** la fiche de connexion (26) comprend une plaque de base (23), à partir de laquelle les broches de connexion (24, 25) dépassent d'une part et sur lesquelles les circuits électriques (21, 22) sont amenés d'autre part.

10. Dispositif selon la revendication 8 ou 9, **caractérisé en ce que** les composants disposés dans le boîtier (2), de préférence une plaque de base présente le cas échéant (23) de la fiche de connexion (26) et/ou l'installation de chauffage (10) sont coincées à l'état monté entre la coque supérieure (4) et la coque inférieure (5).

11. Dispositif selon l'une quelconque des revendications 8 à 10, **caractérisé en ce qu'**un élément décoratif peut être fixé, de préférence encliqueté sur une arête périphérique (28) sur la coque supérieure (4).
